# EUROPÄISCHE PATENTSCHRIFT

(11) **EP 3 649 471 B1**
(45) Veröffentlichungstag und Bekanntmachung des Hinweises auf die Patenterteilung: **29.05.2024**
(21) Anmeldenummer: 18743698.5
(22) Anmeldetag: 04.07.2018
(51) Int. Cl.: G01N 33/543, A61B 5/145, A61M 1/36

(54) **FLUIDANALYSEMODUL SOWIE FLUIDANALYSATOR**
FLUID ANALYSIS MODULE AND FLUID ANALYZER
MODULE D'ANALYSE DE FLUIDE ET ANALYSEUR DE FLUIDE

(30) Priorität: 07.07.2017 DE 102017211693
(43) Veröffentlichungstag der Anmeldung: 13.05.2020
(73) Patentinhaber: ResuSciTec GmbH, 79108 Freiburg (DE)
(72) Erfinder: BENK, Christoph, 79106 Freiburg (DE); GRUDKE, Jürgen, 47839 Krefeld (DE); FELDBRÜGGE, Rainer, 48151 Münster (DE); BORCHARDT, Michael, 48485 Neuenkirchen (DE)
(74) Vertreter: Rösler, Uwe
(86) Internationale Anmeldenummer: PCT/EP2018/068078
(87) Internationale Veröffentlichungsnummer: WO 2019/008026

(56) Entgegenhaltungen:
- US-A- 5 114 580
- US-A1- 2010 137 778
- US-A1- 2015 258 544

## Beschreibung

### Technisches Gebiet

Die Erfindung bezieht sich auf ein Fluidanalysemodul sowie ein Fluidanalysator gemäß beigefügten Anspruchssatz.

### Stand der Technik

Ein gattungsgemäße Fluidanalysemodul ist der DE 10 2011 056 271 A1 zu entnehmen, das sich im Wesentlichen aus drei miteinander verbundene, plattenförmige Funktionseinheiten zusammensetzt, nämlich einer Trägerplatte, einer sog. Fluidkarte sowie einer Sensorkarte. Innerhalb der Trägerplatte sind Vorratskammern sowie ein Aufnahmevolumen enthalten, in denen u. a. eine Sensorflüssigkeit zum Reinigen der Sensoroberfläche bevorratet wird. Die unmittelbar über der Trägerplatte angebrachte Fluidkarte ist fluidisch mit den innerhalb der Trägerplatte vorhandenen Aufnahmevolumen verbunden. Die Fluidkarte enthält eine Vielzahl von Fluidkanälen, in denen Ventile und Pumpen angeordnet sind, vermittels der innerhalb der Trägerplatte bevorratete Fluide sowie auch eine über einen gesonderten Zugang zugeführte Körperflüssigkeit längs bestimmt vorgebbarer Fluidkanäle gefördert werden können. Die mit Sensoren bestückte Sensorkarte schließt einseitig die in der Fluidkarte enthaltenen Fluidkanäle fluiddicht ab und tritt unmittelbar mit den innerhalb den Fluidkanälen geführten Flüssigkeiten in Kontakt. Den Fluidkanälen abgewandt weist die Sensorkarte Sensorkontakte auf, die in elektrischem Kontakt mit einem Messmodul gelangen, an das das Fluidanalysemodul lösbarfest ankoppelbar ist.

Aus der US 6,171,238 B1 ist ein mobiles Handgerät mit einem Biosensor zu entnehmen, der einen amperometrischen Biosensor innerhalb einer Messzelle vorsieht, zu der über ein Schlauchtransportsystem eine, in einem Vorratsbeutel bevorratete Reinigungsflüssigkeit sowie eine über eine Probeöffnung zugebbare Fluidprobe bedarfsweise zuführbar sind. Sämtliche, die Messzelle durchströmenden Fluide gelangen in einen entsprechend vorgesehenen Abfallbeutel. Das mobile Handgerät verfügt über eine Auswerte-, Anzeige- sowie auch Energiespeichereinheit, wodurch ein autarker Betrieb möglich ist.

Die Druckschrift US 4,479,762 A beschreibt ein Modul zur Durchführung einer Plasmapherese, bei der Blut eines Spenders in das Moduls gefördert wird, in dem aus dem Spenderblut Blutplasma separiert wird, und die im Blut verbleibenden roten Blutkörperchen dem Spender wieder rückgeführt werden. Das Modul verfügt über ein Fluidleitungssystem mit Flüssigkeitsreservoirs, das über eine fluiddichte elastische Trennmembran mit einem externen Aktuatorsystem in lösbaren Kontakt bringbar ist, das durch pulsatile Druckwirkung einen Fluidstrom innerhalb des Fluidleitungssystems zu initiieren vermag.

Die Druckschrift US 5,062,774 A beschreibt ein Fluidpumpensystem, an das eine Vielzahl von Flüssigkeitsbehälter anschließbar ist und das beliebige Flüssigkeitsmischungen aus den einzelnen Flüssigkeiten herzustellen vermag.

Allen bekannten Fluidanalysemodulen haftet jedoch der Nachteil an, dass sie aufgrund ihrer Komplexität und optimiert miniaturisierten Integration einer Vielzahl unterschiedlicher Funktionskomponenten technisch anspruchsvolle und somit kostenrelevante Funktionseinheiten darstellen. Insbesondere im Hinblick auf den medizinisch biologischen Einsatz derartiger Fluidanalysemodule zur Untersuchung einer großen Vielzahl unterschiedlicher Messproben, wie bspw. Blutproben von verschiedenen Menschen, werden derartige Fluidanalysemodule zur Vermeidung von Querkontaminationen häufig als Einwegartikel, sog. Disposables ausgebildet.

Die Druckschrift US 2010/13777 A1 beschreibt ein Blut-Analysemodul mit einem Sensor, der mit einem Katheter und einem Spülfüssigkeitsreservoir verbunden ist. Um Blut oder Spülflüssigkeit jeweils alternativ zum Sensor zu führen, ist zwischen zwei Quetschventilen eine peristaltische Rollen-Pumpe positioniert.

Die Druckschrift US 5 114 580 A offenbart eine Hämolyse-Einheit, die über fluidisch miteinander verbundene Reservoire, Pumpen und Drucksensoren verfügt.

Die Druckschrift US 2015/0258544 A1 beschreibt ein mikrofluidisches Analysemodul, das mit einer elastischen Membranschicht bedeckt ist, die durch lokale Deformation eine Absperrventilwirkung längs eines Fluidkanals zu bewirken in der Lage ist.

### Darstellung der Erfindung

Der Erfindung liegt die Aufgabe zugrunde, ein Fluidanalysegerät der vorstehend genannten Gattung in einer möglichst komplexen und kleinbauenden Bauform auszubilden, in der weitgehend sämtliche Komponenten für die Zuführung, Messung und Entsorgung jeweiliger Fluide enthalten sein sollen und die möglichst kostengünstig ausgebildet und für eine einfache Entsorgung, d. h. ohne jedwede elektronische Komponenten, geeignet sein sollen.

Die Lösung der der Erfindung zugrunde liegenden Aufgabe ist im Anspruch 1 angegeben. Gegenstand des Anspruches 10 ist ein Fluidanalysator, der gemäß den Ansprüchen 11 und 12 in bevorzugter Weise für die Analyse von biologischen Flüssigkeiten einsetzbar ist.

Das lösungsgemäße Fluidanalysemodul ist als einheitliche Baueinheit ausgebildet und sieht ein Modulgehäuse vor, das einen Fluideingangsport aufweist. Innerhalb des Modulgehäuses ist wenigstens ein Fluidsensor integriert, der eine mit dem Fluideingangsport in fluidischer Verbindung bringbare Sensoroberfläche aufweist. Ferner ist innerhalb des Modulgehäuses eine Kammer angeordnet, die mit der Sensoroberfläche des wenigstens einen Fluidsensors in fluidische Verbindung bringbar ist. Innerhalb der Kammer ist wenigstens ein erstes Flüssigkeitsreservoir angebracht, das mit der Sensoroberfläche des wenigstens einen Fluidsensors in fluidische Verbindung bringbar ist. Das Modulgehäuse verfügt darüber hinaus über wenigstens eine Modulgehäuseoberfläche, an der zumindest bereichsweise eine elastische, fluiddichte membranartig ausgebildete Trennwand angebracht ist, unter der wenigstens ein fluidisches Funktionselement in Art eines Durchflussventils mit einer Ventilsteuerung sowie wenigstens ein fluidisches Funktionselement in Art einer Förderpumpe angebracht und derart ausgebildet sind, dass die fluidischen Funktionselemente durch ausschließlich lokale mechanische Deformation der Trennwand in wenigstens einer der nachfolgenden Weise betreibbar sind:
- ausschließliches Fördern von Fluid vom Fluideingangsport über die Sensoroberfläche in die Kammer, sowie
- ausschließliches Fördern einer in dem wenigstens einen Flüssigkeitsreservoir bevorrateten Flüssigkeit aus dem Flüssigkeitsreservoirs über die Sensoroberfläche in die Kammer, während das fluidische Funktionselement durch dessen Ventilsteuerung sicherstellt, dass keine fluidische Verbindung zwischen dem Fluideingangsport und der Sensoroberfläche herrscht.

Vorzugsweise ist wenigstens ein zweites Flüssigkeitsreservoir innerhalb der Kammer angebracht ist, das mit der Sensoroberfläche des wenigstens einen Fluidsensors vermittels der wenigstens zwei fluidischen Funktionselemente und/oder wenigstens eines weiteren fluidischen Funktionselementes nach Art eines Durchflussventils oder einer Förderpumpe in fluidische Verbindung bringbar ist. Insbesondere zum Zwecke der Analyse von biologischen Fluiden, allen voran Blut, gilt es die Sensoroberfläche zu reinigen und zu kalibrieren bevor eine Blutmessung durchgeführt wird. So befindet sich in einem ersten, innerhalb der Kammer untergebrachten Flüssigkeitsreservoir eine Sensorspülflüssigkeit oder Kalibrierflüssigkeit und in einem zweiten innerhalb der Kammer untergebrachten Flüssigkeitsreservoir eine Kalibrierflüssigkeit.

Da die Kammer zudem als Auffang- bzw. Entsorgungsvolumen sowohl für das zu vermessende Fluid, bspw. Blut, sowie auch für die in dem wenigstens einen innerhalb der Kammer untergebrachten Flüssigkeitsreservoir bevorrateten Flüssigkeit dient, ist das wenigstens eine Flüssigkeitsreservoir in Form eines fluiddichten elastischen Beutels ausgebildet ist. Bei Entleerung des wenigstens einen Beutels nimmt das Beutelvolumen ab und zugleich nimmt das Aufnahmevolumen für die zu entsorgenden Fluide innerhalb der Kammer zu, die ansonsten über ein konstantes Kammervolumen verfügt.

Das Modulgehäuse ist in einen Messträger- und einen Kammerbereich unterteilbar, d.h. der Messträger sitzt untrennbar auf der Kammer auf und begrenzt diese zumindest einseitig. Dabei weist der Messträger eine der Kammer abgewandte Oberseite aus, an der zumindest bereichsweise die elastische, fluiddichte membranartig ausgebildete Trennwand angebracht ist.

Der Fluideingangsport ist zum einen im Bereich des Messträgers angebracht und innerhalb des Messträgers längs wenigstens einer Fluidleitung fluidisch mit der Sensoroberfläche des wenigstens einen Fluidsensors verbunden, von dem wenigstens eine Fluidleitung innerhalb des Messträgers abführt und in der Kammer mündet. Um die Fluidströmung zwischen dem Fluideingangsport, der Sensoroberfläche und der Kammer zu kontrollieren, ist zum einen längs wenigstens einer der Fluidleitungen wenigstens ein fluidisches Funktionselemente in Art eines Durchflussventils angeordnet, zum anderen sind die die Fluidleitungen mit dem wenigstens einen fluidischen Funktionselement in Art einer Förderpumpe fluidisch in Wirkverbindung bringbar.

Zu Zwecken einer erforderlichen Reinigung oder Kalibrierung der Sensoroberfläche ist das wenigstens eine Flüssigkeitsreservoir über eine in den Bereich des Messträgers einmündende Fluidleitung mit der Sensoroberfläche des wenigstens einen Fluidsensors fluidisch verbunden, von dem wenigstens eine Fluidleitung innerhalb des Messträgers abführt und in die Kammer mündet. Auch in diesem Fall ist längs wenigstens einer dieser Fluidleitungen wenigstens ein fluidisches Funktionselement in Art eines Durchflussventils angeordnet, wobei die Fluidleitungen mit dem wenigstens einem fluidischen Funktionselement in Art einer Förderpumpe fluidisch in Wirkverbindung bringbar sind.

Das wenigstens eine fluidische Funktionselement in Art eines Durchflussventils ist als ein zur Modulgehäuseoberfläche offen ausgebildeter Fluidkanalabschnitt ausgebildet, der von der elastischen, fluiddichten membranartig ausgebildeten Trennwand überspannt ist. Zu Zwecken der Ventilbetätigung bzw. Ventilsteuerung bedarf es einer lokalen, kraftbeaufschlagten Deformation der Trennwand derart, dass der Fluidkanalabschnitt vermittels der lokal deformierten Trennwand lokal fluiddicht abgedichtet ist. Hierzu dient vorzugsweise ein externer Aktor in Form eines Stellwegaktors, der angrenzend zur Trennwand angebracht ist und die Trennwand lokal zu deformieren vermag.

Das wenigstens eine fluidische Funktionselement in Art einer Förderpumpe ist als ein zur Modulgehäuseoberfläche offener Fluidkanalabschnitt ausgebildet, der von der elastischen, fluiddichten membranartig ausgebildeten Trennwand überspannt ist. Zu Zwecken der Pumpenaktivierung bedarf es einer die Trennwand kraftbeaufschlagt lokalen Deformation derart, dass der Fluidkanalabschnitt mittel- oder unmittelbar mit der lokal deformierten Trennwand sich peristaltisch in eine Fluidkanalrichtung fortbewegende Fördervolumina einschließt, vergleichbar dem Prinzip einer Schlauchpumpe.

An der wenigstens einen Modulgehäuseoberfläche sind zudem wenigstens zwei frei zugängliche Elektrodenoberflächen angebracht, die mit dem wenigstens einen Fluidsensor elektrisch verbunden sind.

Sowohl für die mechanische Ansteuerung der fluidischen Funktionselemente als auch für die elektrische Kontaktierung des wenigstens einen Fluidsensors innerhalb des Fluidanalysemoduls bedarf es einer Steuer- und Auswerteeinheit, die an das Fluidanalysemodul lösbar fest derart ankoppelbar ist, dass seitens der Steuer- und Auswerteeinheit vorgesehene mechanische Stellelemente in Eingriff mit den fluidischen Funktionselementen über die elastische, fluiddichte membranartig ausgebildete Trennwand bringbar sind. Ferner ist eine elektrische Verbindung zwischen der Steuer- und Auswerteeinheit und dem wenigstens einen Fluidsensor über die wenigstens zwei, frei an der Modulgehäuseoberfläche angebrachten Elektrodenflächen herstellbar.

Der sich aus dem Fluidanalysemodul und der Steuer- und Auswerteeinheit zusammensetzende Fluidanalysator zeichnet sich dadurch aus, dass sämtliche elektronische Komponenten, die nicht mit dem sensorisch zu vermessenden Fluid in Kontakt treten, seitens der Steuer- und Auswerteeinheit untergebracht sind. Hingegen ist das Fluidanalysemodul gesamtheitlich aus rein mechanischen Komponenten zusammengesetzt und enthält insbesondere bei geeigneter Ausbildung des Fluidsensors keine elektronischen Komponenten.

Der Fluidanalysator eignet sich in besonders vorteilhafter Weise zur sensorischen Analyse von biologischen Flüssigkeiten, bspw. von Blut, Liquor, Serum und Urin. Für diesen Einsatzzweck sind innerhalb der Kammer ein Flüssigkeitsreservoir mit einer Spülflüssigkeit oder Kalibrierflüssigkeit und ein weiteres Flüssigkeitsreservoir mit einer Kalibrierflüssigkeit für die Sensoroberfläche enthalten.

### Kurze Beschreibung der Erfindung

Die Erfindung wird nachstehend ohne Beschränkung des allgemeinen Erfindungsgedankens anhand eines Ausführungsbeispiels unter Bezugnahme auf die Zeichnung exemplarisch beschrieben. Es zeigt:
- Fig. 1: schematischer Aufbau eines lösungsgemäß ausgebildeten Fluidanalysemoduls mit angekoppelter Steuer- und Auswerteeinheit,
- Fig. 2: Illustration eines fluidischen Funktionselements in Form eines Durchflussventils, sowie
- Fig. 3: Illustration eines fluidischen Funktionselements in Form eine Förderpumpe

### Wege zur Ausführung der Erfindung, gewerbliche Verwendbarkeit

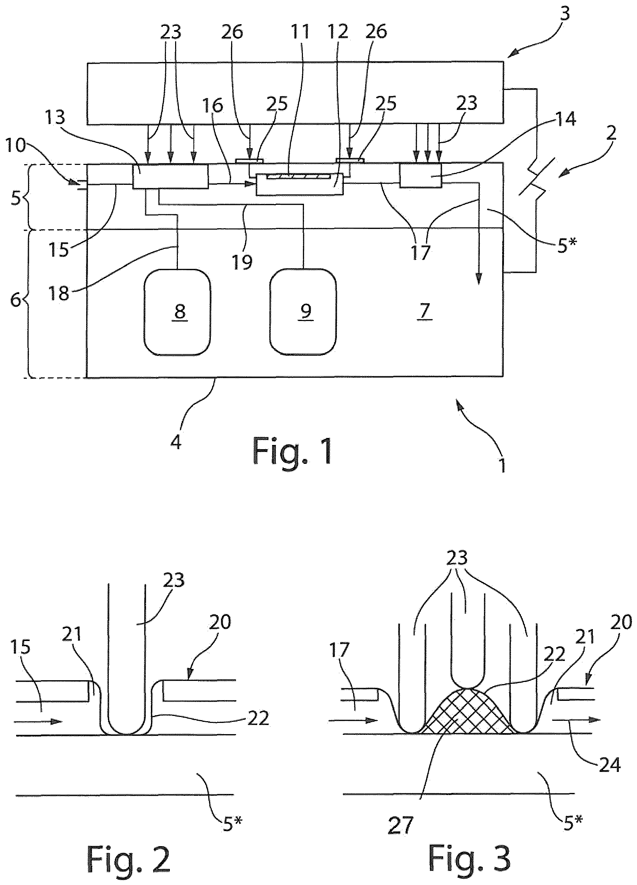

Figur 1 zeigt einen schematischen Aufbau eines Fluidanalysemoduls 1, das über eine mechanisch lösbare Schnittstelle 2 mit einer Steuer- und Auswerteeinheit mechanisch gekoppelt ist. Das Fluidanalysemodul 1 in Verbindung mit der Steuer- und Auswerteeinheit 3 bildet einen Fluidanalysator.

Das in Figur 1 dargestellte Fluidanalysemodul 1 ist von einem Modulgehäuse 4 umgeben und stellt somit eine einheitliche bzw. einheitlich handhabbare Bauform dar. Das Fluidanalysemodul 1 ist funktional unterteilbar in einen Messträgerbereich 5 und einen Kammerbereich 6. Der Kammerbereich 6 weist eine Kammer 7 auf, in der zwei als elastische Beutel ausgebildete Flüssigkeitsreservoire 8, 9 eingebracht sind. Die beutelförmigen Flüssigkeitsreservoire 8, 9 sind mit Ausnahme von in den Messträgerbereich 5 mündenden Fluidleitungen ansonsten lose innerhalb der Kammer 7 gelagert.

Der Messträger 5* verfügt über einen Fluideingangsport 10, über den eine sensorisch zu analysierende Flüssigkeit bzw. ein sensorisch zu analysierendes Fluid, bspw. Blut, in den Messträger 5* einspeisbar ist. Innerhalb des Messträgers 5* befindet sich wenigstens ein über wenigstens eine Sensoroberfläche 11 verfügender Fluidsensor 12, über die innerhalb des Fluidsensors 12 das sensorisch zu untersuchende Fluid geführt wird.

Das über den Fluideingangsport 10 in den Messträger 5* eintretende Fluid wird über die Fluidleitungen 15, 16 in den Fluidsensor 12 geführt. Vom Fluidsensor 12 abführend ist eine Fluidleitung 17 innerhalb des Messträgers 5* vorgesehen, über die das durch den Fluidsensor 12 strömende Fluid unmittelbar in das Volumen der Kammer 7 einmündet, die als Fluidauffang- bzw. Entsorgungskammer dient.

Zur Gewährleistung einer längs der Fluidleitungen 15, 16 und 17 definiert anliegenden Strömung sind innerhalb des Messträgers 5* fluidische Funktionselemente 13 und 14 vorgesehen. Das in Figur 1 zwischen der Fluidleitung 15 und 16 vorgesehene fluidische Funktionselement 13 ist in Art eines Ventils ausgebildet und ermöglicht ein Schließen und Öffnen der Verbindung zwischen den Fluidleitungen 15 und 16. Das längs der Fluidleitung 17 vorgesehene fluidische Funktionselement 14 dient als Förderpumpe und stellt eine kontrollierte Strömung längs der jeweiligen Fluidleitungen sicher. Die fluidischen Funktionselemente 13, 14 können je nach Design an von der Figurendarstellung abweichenden Bereichen längs der Fluidleitungen angebracht sein.

Zum Zwecke der Reinigung oder Kalibrierung der Sensoroberfläche 11 des Fluidsensors 12 wird aus dem Fluidreservoir 8, das eine Spülflüssigkeit oder Kalibrierflüssigkeit bevorratet, Spülflüssigkeit oder Kalibrierflüssigkeit über die Fluidleitungen 18 und 16 zum Fluidsensor 12 geführt, die letztlich wieder über die Fluidleitung 17 in das Kammervolumen der Kammer 7 entsorgt wird. Hierzu gilt es die Ventilsteuerung des fluidischen Funktionselementes 13 derart vorzunehmen, so dass sichergestellt ist, dass die Zuleitung längs der Fluidleitung 15 geschlossen ist und nur die Fluidleitungen 18 und 16 fluidisch verbunden sind.

Zu Zwecken einer Sensorkalibrierung ist innerhalb des Fluidreservoirs 9 eine Kalibrierflüssigkeit bevorratet, die über die Fluidleitungen 19 und 16 zum Fluidsensor 12 gelangt und von diesem über die Fluidleitung 17 in das Auffangvolumen der Kammer 7 überführt wird. Auch in diesem Fall gilt es die Ventilsteuerung des fluidischen Funktionselementes 13 in entsprechender Weise vorzunehmen, d. h. die Fluidleitungen 15 und 18 sind geschlossen, wohingegen die Fluidleitung 19 für einen Zufluss von Kalibrierflüssigkeit zum Fluidsensor 12 geöffnet ist.

In Figur 2 ist ein Querschnitt durch ein fluidisches Funktionselement nach Art eines Ventils 13 illustriert. So weist bspw. die in Figur 2 dargestellte Fluidleitung 15 eine zur Modulgehäuseoberfläche 20 offen ausgebildete Ausnehmung 21 auf, die von einer elastisch fluiddichten, membranartig ausgebildeten Trennwand 22 überspannt ist. Gilt es den Strömungsweg längs des Fluidkanals 15 zu schließen, so bedarf es einer kraftbeaufschlagten Deformation der Trennwand 22 derart, so dass sie für einen lokalen fluiddichten Abschluss innerhalb der Fluidleitung 15 sorgt. In vorteilhafter Weise dient hierzu ein Stellelement 23, das zu Seiten der Steuer- und Auswerteeinheit 3 angebracht ist.

Wenigstens längs jeder der in Figur 1 dargestellten Fluidkanäle 15, 18 und 19 ist jeweils ein gemäß Figur 2 ausgebildetes fluidisches Funktionselement vorgesehen.

Figur 3 illustriert eine mögliche Ausführungsform zur Realisierung eines fluidischen Funktionselementes in Art einer Förderpumpe. Es sei angenommen, dass der Fluidkanal 17 eine zur Modulgehäuseoberfläche 20 orientierte Ausnehmung 21 aufweist, die von einer elastischen, fluiddichten membranartig ausgebildeten Trennwand 22 überspannt ist. Zum Zwecke der Einprägung einer Fluidströmung in Förderrichtung 24 treten wenigstens drei zu Seiten der Steuer- und Auswerteeinheit 3 angebrachte Stellelemente 23 mit jeweils aufeinander abgestimmten Stellweghüben in Kontakt mit der Trennwand 22, die es derart zu deformieren gilt, so dass sie mit der Fluidkanalwand ein sich peristaltisch in Förderrichtung 24 fortbewegendes Fördervolumen 27 einschließt.

Schließlich sind an der Modulgehäuseoberfläche 20 wenigstens zwei Elektrodenoberflächen 25 angebracht, über die die Fluidsensorsignale abgegriffen werden können. Hierzu weist die Steuer- und Auswerteeinheit 3 entsprechende Elektrodenkontakte 26 auf.

Das lösungsgemäße Fluidanalysemodul 1 besteht aus einfach und kostengünstig zu realisierenden Komponenten und eignet sich in besonderer Weise als Einwegartikel bzw. Disposable. Da das Fluidanalysemodul 1 keine elektrischen Komponenten beinhaltet, ist die Entsorgung unproblematisch. Sämtliche Aktoren und elektronische Komponenten sind an der Steuer- und Auswerteeinheit 3 angebracht und treten ausschließlich über eine fluiddichte Trennwand mit dem Fluidanalysator in Wirkverbindung, d. h. die Steuer- und Auswerteeinheit erfährt keine Kontaminationen während eines Messvorgangs bzw. Messzyklus.

### Bezugszeichenliste

- 1: Fluidanalysemodul
- 2: mechanische Schnittstelle
- 3: Steuer- und Auswerteeinheit
- 4: Modulgehäuse
- 5: Messträgerbereich
- 6: Kammerbereich
- 7: Kammer
- 8: erstes Fluidreservoir
- 9: zweites Fluidreservoir
- 10: Fluideingangsportal
- 11: Sensoroberfläche
- 12: Fluidsensor
- 13: fluidisches Funktionselement in Art eines Durchflussventils
- 14: fluidisches Funktionselement in Art einer Förderpumpe
- 15, 16, 17, 18, 19: Fluidleitungen
- 20: Modulgehäuseoberfläche
- 21: Ausnehmung
- 22: Trennwand
- 23: Stellelement
- 24: Förderrichtung
- 25: Elektrodenoberflächen
- 26: Elektrodenkontakte
- 27: Fördervolumina

## Patentansprüche

1. Fluidanalysemodul (1) mit
- einem Modulgehäuse (4) mit einem Fluideingangsport (10),
- wenigstens einem innerhalb des Modulgehäuses (4) integrierten Fluidsensor (12) mit einer mit dem Fluideingangsport (10) in fluidischer Verbindung bringbaren Sensoroberfläche (11),
- einer innerhalb des Modulgehäuses (4) integrierten Kammer (7), die mit der Sensoroberfläche (11) des wenigstens einen Fluidsensors (12) in fluidische Verbindung bringbar ist,
- wenigstens einem innerhalb der Kammer (7) angebrachten ersten Flüssigkeitsreservoir (8), das mit der Sensoroberfläche (11) des wenigstens einen Fluidsensors (12) in fluidische Verbindung bringbar ist, sowie
- wenigstens einer Modulgehäuseoberfläche (20), an der zumindest bereichsweise eine elastische, fluiddichte membranartig ausgebildete Trennwand (22) angebracht ist, unter der wenigstens ein fluidisches Funktionselement in Art eines Durchflussventils (13) mit einer Ventilsteuerung sowie wenigstens ein fluidisches Funktionselement in Art einer Förderpumpe (14) angebracht und derart ausgebildet sind, dass die fluidischen Funktionselemente (13, 14) durch ausschließlich lokale mechanische Deformation der Trennwand in wenigstens einer der nachfolgenden Weise betreibbar sind:
- ausschließliches Fördern von Fluid vom Fluideingangsport über die Sensoroberfläche (11) in die Kammer (7), sowie
- ausschließliches Fördern einer in dem wenigstens einen Flüssigkeitsreservoir (8) bevorrateten Flüssigkeit aus dem Flüssigkeitsreservoirs (8) über die Sensoroberfläche (11) in die Kammer (7), während das fluidische Funktionselement (13) durch dessen Ventilsteuerung sicherstellt, dass keine fluidische Verbindung zwischen dem Fluideingangsport (10) und der Sensoroberfläche (11) herrscht.

2. Fluidanalysemodul nach Anspruch 1,
**dadurch gekennzeichnet, dass** wenigstens ein zweites Flüssigkeitsreservoir (9) innerhalb der Kammer (7) angebracht ist, das mit der Sensoroberfläche (11) des wenigstens einen Fluidsensors (12) vermittels der wenigstens zwei fluidischen Funktionselemente (13, 14) und/oder wenigstens eines weiteren fluidischen Funktionselementes nach Art eines Durchflussventils oder einer Förderpumpe in fluidische Verbindung bringbar ist.

3. Fluidanalysemodul nach Anspruch 1 oder 2,
**dadurch gekennzeichnet, dass** das wenigstens eine Flüssigkeitsreservoir (8) in Form eines fluiddichten elastischen Beutels ausgebildet ist.

4. Fluidanalysemodul nach einem der Ansprüche 1 bis 3,
**dadurch gekennzeichnet, dass** das Modulgehäuse (4) in einen Messträger- (5) und einen Kammerbereich (6) unterteilbar ist,
dass der Messträger (5) untrennbar auf der Kammer (7) aufsitzt und diese zumindest einseitig begrenzt,
dass der Messträger (5) eine der Kammer (7) abgewandte Oberseite aufweist, an der zumindest bereichsweise die elastische, fluiddichte membranartig ausgebildete Trennwand (22) angebracht ist.

5. Fluidanalysemodul nach Anspruch 4,
**dadurch gekennzeichnet, dass** der Fluideingangsport (10) im Bereich des Messträgers (5) angebracht ist und innerhalb des Messträgers (5) längs wenigstens einer Fluidleitung (15, 16) fluidisch mit der Sensoroberfläche (11) des wenigstens einen Fluidsensors (12) verbunden ist, von dem wenigstens eine Fluidleitung (17) innerhalb des Messträgers (5) abführt und in die Kammer (7) mündet, und
dass längs wenigstens einer der Fluidleitungen (15, 16, 17) wenigstens eines der fluidischen Funktionselemente in Art eines Durchflussventils (13) angeordnet ist, und dass die Fluidleitungen (17) mit dem wenigstens einen fluidischen Funktionselement in Art einer Förderpumpe (14) fluidisch in Wirkverbindung bringbar sind.

6. Fluidanalysemodul nach Anspruch 4 oder 5,
**dadurch gekennzeichnet, dass** das wenigstens eine Flüssigkeitsreservoir (8, 9) über eine in den Bereich des Messträgers (5) einmündende Fluidleitung (18, 19) mit der Sensoroberfläche (11) des wenigstens einen Fluidsensors (12) fluidisch verbunden ist, von dem wenigstens eine Fluidleitung (17) innerhalb des Messträgers (5) abführt und in die Kammer (7) mündet, und
dass längs wenigstens einer der Fluidleitungen (18, 19) wenigstens eines der fluidischen Funktionselemente in Art eines Durchflussventils (13) angeordnet ist, und dass die Fluidleitungen (18, 19) mit dem wenigstens einen fluidischen Funktionselement in Art einer Förderpumpe (14) fluidisch in Wirkverbindung bringbar sind.

7. Fluidanalysemodul nach einem der Ansprüche 1 bis 6,
**dadurch gekennzeichnet, dass** an wenigstens einer Modulgehäuseoberfläche (20) wenigstens zwei frei zugängliche Elektrodenoberflächen (25) angebracht sind, die mit dem wenigstens einen Fluidsensor (12) elektrisch verbunden sind.

8. Fluidanalysemodul nach einem der Ansprüche 1 bis 7,
**dadurch gekennzeichnet, dass** das wenigstens eine fluidische Funktionselement in Art eines Durchflussventils (13) ein zur Modulgehäuseoberfläche (20) offen ausgebildeter Fluidkanalabschnitt ist, der von der elastischen, fluiddichten membranartig ausgebildeten Trennwand (22) überspannt ist, und
dass die Trennwand (20) kraftbeaufschlagt lokal derart deformierbar ist, dass der Fluidkanalabschnitt vermittels der lokal deformierten Trennwand (22) lokal fluiddicht abgedichtet ist.

9. Fluidanalysemodul nach einem der Ansprüche 1 bis 8,
**dadurch gekennzeichnet, dass** das wenigstens eine fluidische Funktionselement in Art eine Förderpumpe (14) einen zur Modulgehäuseoberfläche (20) offen ausgebildeten Fluidkanalabschnitt ist, der von der elastischen, fluiddichten membranartig ausgebildeten Trennwand (22) überspannt ist, und
dass die Trennwand (22) kraftbeaufschlagt lokal derart deformierbar ist, dass der Fluidkanalabschnitt mittel- oder unmittelbar mit der lokal deformierten Trennwand (22) sich peristaltisch in eine Fluidkanalrichtung fortbewegende Fördervolumina (27) einschließt.

10. Fluidanalysator mit einem Fluidanalysemodul nach einem der Ansprüche 1 bis 9,
**dadurch gekennzeichnet, dass** eine Steuer- und Auswerteeinheit (3) vorgesehen ist, an die das Fluidanalysemodul (1) lösbar fest derart ankoppelbar ist, dass seitens der Steuer- und Auswerteeinheit (3) vorgesehene mechanische Stellelemente in Eingriff mit den fluidischen Funktionselementen über die elastische, fluiddichte membranartig ausgebildete Trennwand (22) bringbar sind, und
dass eine elektrische Verbindung zwischen der Steuer- und Auswerteeinheit (3) und dem wenigstens einen Fluidsensor (12) über wenigstens zwei frei an der Modulgehäuseoberfläche (20) angebrachten Elektrodenflächen (25) herstellbar ist.

11. Verwendung ex-vivo des Fluidanalysemoduls nach einem der Ansprüche 1 bis 9 als Analysator für biologische Flüssigkeiten, umfassend, Blut, Liquor, Serum, Urin, Reperfusat.

12. Verwendung nach Anspruch 11,
**dadurch gekennzeichnet, dass** innerhalb der Kammer (7) ein Flüssigkeitsreservoir (8) mit einer Spülflüssigkeit oder Kalibrierflüssigkeit und ein weiteres Flüssigkeitsreservoir (9) mit einer Kalibrierflüssigkeit enthalten sind.

## Claims

1. A fluid analysis module (1) comprising
- a module housing (4) with a fluid inlet port (10),
- at least one fluid sensor (12) integrated within the module housing (4) and having a sensor surface (11) that can be brought into a fluidic connection with the fluid inlet port (10),
- a chamber (7) integrated within the module housing (4), which chamber can be brought into a fluidic connection with the sensor surface (11) of the at least one fluid sensor (12),
- at least one first liquid reservoir (8), which is attached within the chamber (7) and which can be brought into a fluidic connection with the sensor surface (11) of the at least one fluid sensor (12), and
- at least one module housing surface (20), on which an elastic, fluid-tight, membrane-like separating wall (22) is attached at least in regions, under which at least one fluidic function element in the manner of a flow valve (13) with a valve control and also at least one fluidic function element in the manner of a delivery pump (14) are attached and formed in such a way that the fluidic function elements (13, 14) are operable by exclusively local mechanical deformation of the separating wall in at least one of the following ways:
- only fluid from the fluid inlet port is conveyed into the chamber (7) via the sensor surface (11), and
- only a liquid stored in the at least one liquid reservoir (8) is conveyed from the liquid reservoir (8) into the chamber (7) via the sensor surface (11), whilst the fluidic function element (13), through its valve control, ensures that no fluidic connection prevails between the fluid inlet port (10) and the sensor surface (11).

2. The fluid analysis module according to claim 1,
**characterised in that** at least one second liquid reservoir (9) is attached inside the chamber (7) and can be brought into a fluidic connection with the sensor surface (11) of the at least one fluid sensor (12) by means of the at least two fluidic function elements (13, 14) and/or at least one further fluidic function element in the manner of a flow valve or a delivery pump.

3. The fluid analysis module according to claim 1 or 2, **characterised in that** the at least one liquid reservoir (8) is formed as a fluid-tight elastic bag.

4. The fluid analysis module according to one of claims 1 to 3,
**characterised in that** the module housing (4) can be subdivided into a measuring carrier region (5) and a chamber region (6),
**in that** the measuring carrier (5) sits inseparably on the chamber (7) and delimits the latter at least on one side,
**in that** the measuring carrier (5) has an upper face remote from the chamber (7) and the elastic fluid-tight, membrane-like separating wall (22) is attached at least in portions to said upper face.

5. The fluid analysis module according to claim 4,
**characterised in that** the fluid inlet port (10) is attached in the region of the measuring carrier (5) and is fluidically connected within the measuring carrier (5) along at least one fluid line (15, 16) to the sensor surface (11) of the at least one fluid sensor (12), from which at least one fluid line (17) leads within the measuring carrier (5) and opens out into the chamber (7), and **in that** at least one of the fluidic function elements is arranged in the manner of a flow valve (13) along at least one of the fluid lines (15, 16, 17), and **in that** the fluid lines (17) can be brought fluidically into operative connection with the at least one fluidic function element in the manner of a delivery pump (14).

6. The fluid analysis module according to claim 4 or 5,
**characterised in that** the at least one liquid reservoir (8, 9) is fluidically connected to the sensor surface (11) of the at least one fluid sensor (12) via a fluid line (18, 19) opening out into the region of the measuring carrier (5), with at least one fluid line (17) discharging from said sensor surface within the measuring carrier (5) and opening out into the chamber (7), and
**in that** at least one of the fluidic function elements is arranged in the manner of a flow valve (13) along at least one of the fluid lines (18, 19), and
**in that** the fluid lines (18, 19) can be brought fluidically into operative connection with the at least one fluidic function element in the manner of a delivery pump (14).

7. The fluid analysis module according to any one of claims 1 to 6,
**characterised in that** at least two freely accessible electrode surfaces (25), which are electrically connected to the at least one fluid sensor (12), are attached to at least one module housing surface (20).

8. The fluid analysis module according to any one of claims 1 to 7,
**characterised in that** the at least one fluidic function element in the form of a flow valve (13) is a fluid channel portion which opens toward the module housing surface (20) and which is spanned by the elastic fluid-tight, membrane-like separating wall (22), and
**in that** the separating wall (20) is locally deformable by application of force thereto, in such a way that the fluid channel portion is sealed locally, fluid-tightly by means of the locally deformed separating wall (22).

9. The fluid analysis module according to any one of claims 1 to 8,
**characterised in that** the at least one fluidic function element in the manner of a delivery pump (14) is a fluid channel portion which opens towards the at least one module housing surface (20) and which is spanned by the elastic, fluid-tight, membrane-like separating wall (22), and
**in that** the separating wall (22) is locally deformable by the application of force, in such a way that the fluid channel portion encloses peristaltically, indirectly or directly with the locally deformed separating wall (22), delivery volumes (27) moving forward in a fluid channel direction.

10. A fluid analyser comprising a fluid analysis module according to any one of claims 1 to 9,
**characterised in that** a control and evaluation unit (3) is provided, to which the fluid analysis module (1) can be coupled releasably and fixedly in such a way that mechanical actuating elements provided on the control and evaluation unit (3) can be brought into engagement with the fluidic function elements via the elastic, fluid-tight, membrane-like separating wall (22), and
**in that** an electrical connection can be produced between the control and evaluation unit (3) and the at least one fluid sensor (12) via at least two electrode surfaces (25) attached freely to the module housing surface (20).

11. Use ex-vivo of the fluid analysis module according to any one of claims 1 to 9 as an analyser for biological liquids, comprising blood, liquor, serum, urine or reperfusate.

12. The use according to claim 11,
**characterised in that** a liquid reservoir (8) with a flushing liquid or calibration liquid and a further liquid reservoir (9) with a calibration liquid are contained within the chamber (7).

## Revendications

1. Module d'analyse de fluides (1), comprenant
- un boîtier de module (4), pourvu d'un port d'entrée (10) de fluide,
- au moins un détecteur de fluide (12), intégré à l'intérieur du boîtier de module (4), pourvu d'une surface de détecteur (11), susceptible d'être amenée en liaison fluidique avec le port d'entrée (10) de fluide,
- une chambre (7) intégrée à l'intérieur du boîtier de module (4), qui est susceptible d'être amenée en liaison fluidique avec la surface de détecteur (11) de l'au moins un détecteur de fluide (12),
- au moins un premier réservoir de fluide (8), monté à l'intérieur de la chambre (7), qui est susceptible d'être amené en liaison fluidique avec la surface de détecteur (11) de l'au moins un détecteur de fluide (12), ainsi qu'au moins une surface (20) de boîtier de module, sur laquelle est montée au moins par endroits une paroi de séparation (22) élastique, étanche au fluide, conçue à la manière d'une membrane, sous laquelle au moins un élément fonctionnel fluidique, du type d'une vanne de débit (13), pourvue d'une commande de vanne, ainsi qu'au moins un élément fonctionnel fluidique, du type d'une pompe d'alimentation (14) sont montés et conçus de telle sorte que les éléments fonctionnels (13, 14) fluidiques soient exploitables exclusivement par déformation mécanique locale de la paroi de séparation, d'au moins l'une des manières suivantes :
- transport exclusif de fluide du port d'entrée de fluide via la surface de détecteur (11) dans la chambre (7), et
- transport exclusif d'un liquide stocké dans l'au moins un réservoir de fluide (8) hors du réservoir de fluide (8) via la surface de détecteur (11) dans la chambre (7), alors que l'élément fonctionnel (13) fluidique assure du fait de sa commande de vanne qu'il n'existe aucune liaison fluidique entre le port d'entrée (10) de fluide et la surface de détecteur (11).

2. Module d'analyse de fluides selon la revendication 1, **caractérisé en ce qu'**au moins un deuxième réservoir de fluide (9) est monté à l'intérieur de la chambre (7), lequel est susceptible d'être amené en liaison fluidique avec la surface de détecteur (11) de l'au moins un détecteur de fluide (12) par l'intermédiaire des au moins deux éléments fonctionnels (13, 14) fluidiques et / ou d'au au moins un éléments fonctionnel fluidique additionnel, du type d'une vanne de débit ou d'une pompe d'alimentation in.

3. Module d'analyse de fluides selon la revendication 1 ou 2,
**caractérisé en ce que** l'au moins un réservoir de fluide (8) est conçu sous la forme d'un sachet élastique étanche au fluide.

4. Module d'analyse de fluides selon l'une quelconque des revendications 1 à 3,
**caractérisé en ce que** le boîtier de module (4) est divisible en une zone de support de mesurage (5) et une zone de chambre (6),
**en ce que** le support de mesurage (5) repose de manière non désolidarisable sur la chambre (7) et délimite cette dernière au moins d'un côté,
**en ce que** le support de mesurage (5) comporte une face supérieure opposée à la chambre (7), sur laquelle est montée au moins par endroits la paroi de séparation (22) élastique, étanche au fluide, conçue à la manière d'une membrane.

5. Module d'analyse de fluides selon la revendication 4,
**caractérisé en ce que** le port d'entrée (10) de fluide est monté dans la zone du support de mesurage (5) et est en liaison fluidique à l'intérieur du support de mesurage (5), le long d'au moins une conduite de fluide (15, 16) avec la surface de détecteur (11) de l'au moins un détecteur de fluide (12), à partir duquel part au moins une conduite de fluide (17) à l'intérieur du support de mesurage (5) et débouche dans la chambre (7) et
**en ce que** le long d'au moins l'une des conduites de fluide (15, 16, 17), est placé au moins l'un des éléments fonctionnels fluidiques du type d'une vanne de débit (13) et **en ce que** les conduites de fluide (17) sont susceptibles d'être amenées en liaison fluidique active avec l'au moins un élément fonctionnel fluidique, du type d'une pompe d'alimentation (14).

6. Module d'analyse de fluides selon la revendication 4 ou 5,
**caractérisé en ce que** l'au moins un réservoir de fluide (8, 9) est en liaison fluidique via une conduite de fluide (18, 19) débouchant dans la zone du support de mesurage (5) avec la surface de détecteur (11) de l'au moins un détecteur de fluide (12), à partir duquel part au moins un conduite de fluide (17) à l'intérieur du support de mesurage (5) et débouche dans la chambre (7) et
**en ce que** le long d'au moins l'une des conduites de fluide (18, 19), est placé au moins l'un des éléments fonctionnels fluidiques du type d'une vanne de débit (13) et **en ce que** les conduites de fluide (18, 19) sont susceptibles d'être amenées en liaison fluidique active avec l'au moins un élément fonctionnel fluidique, du type d'une pompe d'alimentation (14).

7. Module d'analyse de fluides selon l'une quelconque des revendications 1 à 6,
**caractérisé en ce que** sur au moins une surface (20) de boîtier de module sont montées au moins deux surfaces d'électrode (25) librement accessibles qui sont connectées électriquement avec l'au moins un détecteur de fluide (12).

8. Module d'analyse de fluides selon l'une quelconque des revendications 1 à 7,
**caractérisé en ce que** l'au moins un élément fonctionnel fluidique du type d'une vanne de débit (13) est un segment de canal de fluide conçu en étant ouvert sur la surface (20) de boîtier de module qui est enjambé par la paroi de séparation (22) élastique, étanche au fluide, conçue à la manière d'une membrane et
**en ce que** sous l'effet d'une force, la paroi de séparation (20) est localement déformable, de telle sorte que l'étanchéité locale au fluide du segment de canal de fluide soit assurée par l'intermédiaire de la paroi de séparation (22) localement déformée.

9. Module d'analyse de fluides selon l'une quelconque des revendications 1 à 8,
**caractérisé en ce que** l'au moins un élément fonctionnel fluidique du type d'une pompe d'alimentation (14) est un segment de canal de fluide conçu en étant ouvert sur la surface (20) de boîtier de module qui est enjambé par la paroi de séparation (22) élastique, étanche au fluide, conçue à la manière d'une membrane,
**en ce que** sous l'effet d'une force, la paroi de séparation (22) est localement déformable, de telle sorte que le segment de canal de fluide s'inclue indirectement ou directement de manière péristaltique avec la paroi de séparation (22) localement déformée dans des volumes transportés (27) se déplaçant dans une direction du canal de fluide.

10. Analyseur de fluides, doté d'un module d'analyse de fluides selon l'une quelconque des revendications 1 à 9,
**caractérisé en ce qu'**il est prévu une unité de commande et d'évaluation (3), à laquelle le module d'analyse de fluides (1) peut s'accoupler solidement de manière amovible, de telle sorte que des éléments de réglage mécaniques prévus du côté de l'unité de commande et d'évaluation (3) soient susceptibles d'être amenés en engagement dans les éléments fonctionnels fluidique via la paroi de séparation (22) élastique, étanche au fluide, conçue à la manière d'une membrane et
**en ce qu'**une connexion électrique est susceptible d'être établie entre l'unité de commande et d'évaluation (3) et l'au moins un détecteur de fluide (12) via deux surfaces d'électrode (25) montées librement sur la surface (20) de boîtier de module.

11. Utilisation ex-vivo du module d'analyse de fluides selon l'une quelconque des revendications 1 à 9 en tant qu'analyseur de liquides biologiques, incluant le sang, le liquide céphalo-rachidien, le sérum, l'urine, la reperfusion.

12. Utilisation selon la revendication 11,
**caractérisée en ce qu'**à l'intérieur de la chambre (7) est contenu un réservoir de fluide (8), doté d'un liquide de rinçage ou d'un liquide d'étalonnage et d'un réservoir de fluide (9) additionnel, doté d'un liquide d'étalonnage.
